(19) Europäisches Patentamt
European Patent Office
Office européen des brevets

(11) Publication number: 0 469 262 B1

(12) EUROPEAN PATENT SPECIFICATION

(45) Date of publication of patent specification: 22.02.95

(51) Int. Cl.[6]: C07D 207/34, C07D 207/36, A01N 43/36, C07C 21/18

(21) Application number: 91108535.5

(22) Date of filing: 25.05.91

# (54) Process for the preparation of insecticidal acaridical and nematicidal 2-aryl-5-(trifluoromethyl) pyrrole compounds.

(30) Priority: 31.07.90 US 560396
31.07.90 US 560403

(43) Date of publication of application:
05.02.92 Bulletin 92/06

(45) Publication of the grant of the patent:
22.02.95 Bulletin 95/08

(84) Designated Contracting States:
AT BE CH DE DK ES FR GB GR IT LI LU NL SE

(56) References cited:
EP-A- 0 312 723
EP-A- 0 347 488
EP-A- 0 358 047

J. ORG. CHEM. vol. 43, no. 22, 19 December 1977, pages 4273 - 4276; ALBONICOET AL: '2-Chloroacrylonitrile as a Cyclodipolarophile in 1,3-Cycloadditions. 3-Cyanopyrroles'

(73) Proprietor: AMERICAN CYANAMID COMPANY
One Cyanamid Plaza
Wayne, NJ 07470-8426 (US)

(72) Inventor: Addor, Roger Williams
97 Woosamonsa Road
Pennington,
New Jersey 00834 (US)
Inventor: Furch, Joseph Augustus
68 North Brook Avenue
Lawrenceville,
New Jersey 08648 (US)
Inventor: Kuhn, David George
2 Adrian Place
Newtown,
Pennsylvania 18940 (US)
Inventor: Kremer, Kenneth Alfred Martin
B-15, Carver Place
Lawrenceville,
New Jersey 08648 (US)

(74) Representative: Wächtershäuser, Günter, Prof. Dr.
Patentanwalt
Tal 29
D-80331 München (DE)

EP 0 469 262 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

It is an object of the present invention to provide a more efficient process for the preparation of insecticidal, nematicidal and acaricidal 2-aryl-5-(trifluoromethyl)pyrrole compounds which reduces excess starting materials and reaction time and increases product yield.

It is a further object of the invention to provide an efficient method of preparing the 1,1,1-trifluoro-2-propene compounds of the present invention.

The reaction of 2-chloroacrylonitrile with N-alkyl-alpha-amino acids in acetic anhydride to yield 3-cyanopyrroles is known (J.Org.Chem., Vol. 43, no. 22, pages 4273-4276). EP-A-0 347 488 relates to certain arylpyrrole insecticidal agents and a method for the preparation thereof but does not disclose the inventive method of the present invention. EP-A-0 358 047 discloses a method of controlling phytopathogenic fungi using certain arylpyrrole compounds. EP-A-0 312 723 discloses a method for controlling mollusks and bait compositions containing molluscicidally amount of certain arylpyrrole compounds. None of the cited art discloses the process of the present invention.

### SUMMARY OF THE INVENTION

The present invention relates to a process for the preparation of insecticidal, nematicidal and acaricidal 2-aryl-5-(trifluoromethyl)pyrrole compounds of formula I

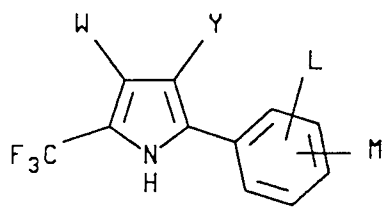

(I)

wherein

| | |
|---|---|
| W is | $C_1$-$C_4$ alkyl, $CF_3$ or H; |
| Y is | CN, $NO_2$ or $CO_2R$; |
| R is | $C_1$-$C_4$ alkyl; |
| L is | H, F, Cl or Br; |
| M is | H, F, Cl, Br, I, $CF_3$, $NO_2$ or $OR_1$; and |
| $R_1$ is | $C_1$-$C_3$ alkyl or $C_2F_4H$ which comprises reacting a second compound having the structure |

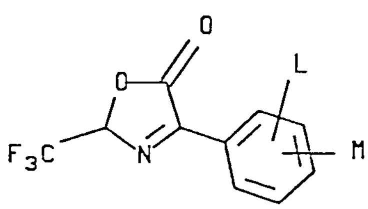

(II)

wherein L and M are as described above with at least 1 molar equivalent of a third compound having the structure

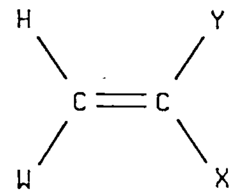

(III)

wherein W and Y are as described above, X is Cl, Br, I or

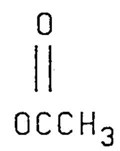

and the cis and trans isomers thereof in the presence of at least 1 molar equivalent of a base selected from an alkali metal carbonate, $C_1$-$C_4$ trialkylamine or pyridine and a polar solvent selected from acetonitrile, dimethylformamide, dimethylsulfoxide, ethanol, methanol or isopropanol, to form said first compound.

## DETAILED DESCRIPTION OF THE INVENTION

The present invention provides a process for the preparation in good yield of insecticidal, nematicidal and acaricidal compounds of formula I

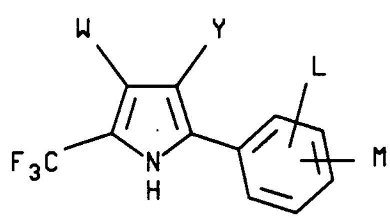

(I)

wherein

- W is $C_1$-$C_4$ alkyl, $CF_3$ or H;
- Y is CN, $NO_2$ or $CO_2R$;
- R is $C_1$-$C_4$ alkyl;
- L is H, F, Cl or Br;
- M is H, F, Cl, Br, I, $CF_3$, $NO_2$ or $OR_1$; and
- $R_1$ is $C_1$-$C_3$ alkyl or $C_2F_4H$ by an efficient single step reaction between an azalactone compound of formula II

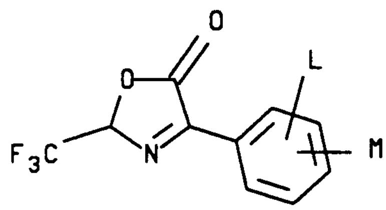

(II)

wherein L and M are as described above and an $\alpha$-halo-$\alpha,\beta$-unsaturated nitrile, ester or nitro compound of formula III

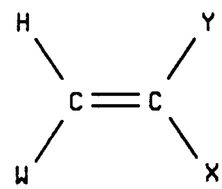

(III)

wherein W and Y are as described above; X is Cl, Br, I or

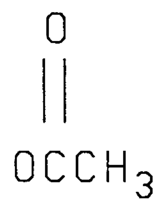

and the cis and trans isomers thereof in the presence of a polar solvent and a base.

The process comprises reacting a formula II azalactone as described above with at least one molar equivalent, preferably one to five molar equivalents, of a formula III $\alpha$-halo-$\alpha,\beta$-unsaturated nitrile, ester or nitro compound as described above and at least about one molar equivalent, preferably about one to five molar equivalents, of a base in the presence of a polar solvent preferably at a temperature range of 20°C to 180°C to form 2-aryl-5-(trifluoromethyl)pyrrole compounds of formula I. Naturally, it is especially preferred to use as little excess reactants as possible. Advantageously, stoichiometric quantities may be used in the present invention and still maintain good yields.

The product formula I compounds may be isolated by conventional techniques such as dilution of the reaction mixture with water and filtration of the formula I product or extraction of said product with a suitable solvent. In the isolation procedure any suitable extraction solvents may be employed, including water-immiscible solvents such as ether, ethyl acetate, methylene chloride and the like.

Bases suitable for use in the process of the invention include bases such as alkali metal carbonates, $C_1$-$C_4$ trialkylamines, alkali metal hydroxides and pyridine. Preferred bases are pyridine, triethylamine and sodium carbonate.

Reaction solvents suitable for use in the above process include any polar solvents, for example solvents such as acetonitrile, dimethylsulfoxide, dimethylformamide, ethanol, methanol and isopropanol. Acetonitrile and dimethylformamide are preferred reaction solvents.

Certain starting formula III compounds are described below.

The 1,1,1-trifluoro-2-propene compounds of the present invention have the structural formula IIIa:

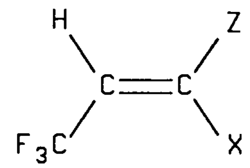

(IIIa)

wherein

X is Cl, Br or I;

Z is CN, or $CO_2R$;

R is $C_1$-$C_4$ alkyl; and the cis and trans isomers thereof.

Preferred 1,1,1-trifluoro-2-propene compounds of the invention are those in which X is Cl or Br; Z is CN.

Formula IIIa compounds may be prepared by reacting trifluoroacetaldehyde with (triphenylphosphoranylidene)acetonitrile in a solvent to give 4,4,4-trifluorocrotononitrile. The said 4,4,4-trifluorocrotononitrile or other compound having the structural formula IV:

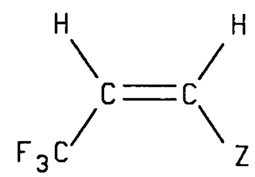

(IV)

wherein

Z is CN, or $CO_2R$;

R is $C_1$-$C_4$ alkyl; and the cis and trans isomers thereof;

is reacted with at least two molar equivalents, preferably two to five molar equivalents of a halogenating agent in the presence of a solvent to form 2,3-dihalo-1,1,1-trifluoropropane compounds of formula V:

$F_3CCHXCHXZ$ (V)

wherein

X is Cl, Br or I; and Z is as described above.

Halogenating agents that may be employed include bromine, chlorine, iodine, and the like. Reaction solvents suitable for use in the above-described reaction include tetrahydrofuran, carbon tetrachloride and the like. The resultant 2,3-dihalo-1,1,1,-trifluoropropane is then reacted with at least one molar equivalent, preferably one to three molar equivalents, of a base in the presence of a solvent to form 1,1,1-trifluoro-2-propene compounds of formula I. Bases suitable for use in the reaction include triethylamine, pyridine and sodium carbonate and the like. Solvents such as ether and tetrahydrofuran may be preferably employed. This reaction scheme is illustrated as follows:

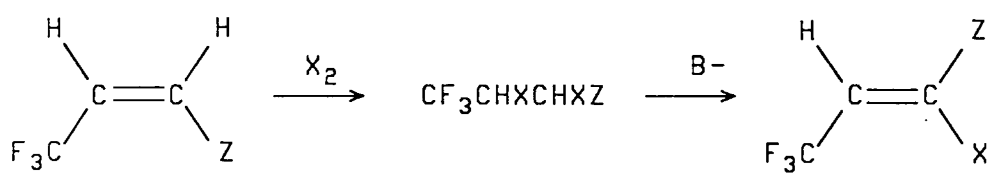


(IV) (V) (IIIa)

The preparation of formula IIIa compounds was illustrated in the previous reaction scheme, except when R is hydrogen. To prepare the formula IIIa compounds when R is hydrogen an additional hydrolysis step may be required. Hydrolysis of a formula IIIa compound when R is not hydrogen yields another formula IIIa compound having the same structure except that R is now hydrogen.

In order to facilitate a further understanding of the invention, the following examples are presented to illustrate more specific details thereof. The invention is not to be limited thereby except as defined in the claims.

**EXAMPLE 1**

**Preparation of 2-Phenyl-5-(trifluoromethyl)pyrrole-3-carbonitrile in the presence of a base.**

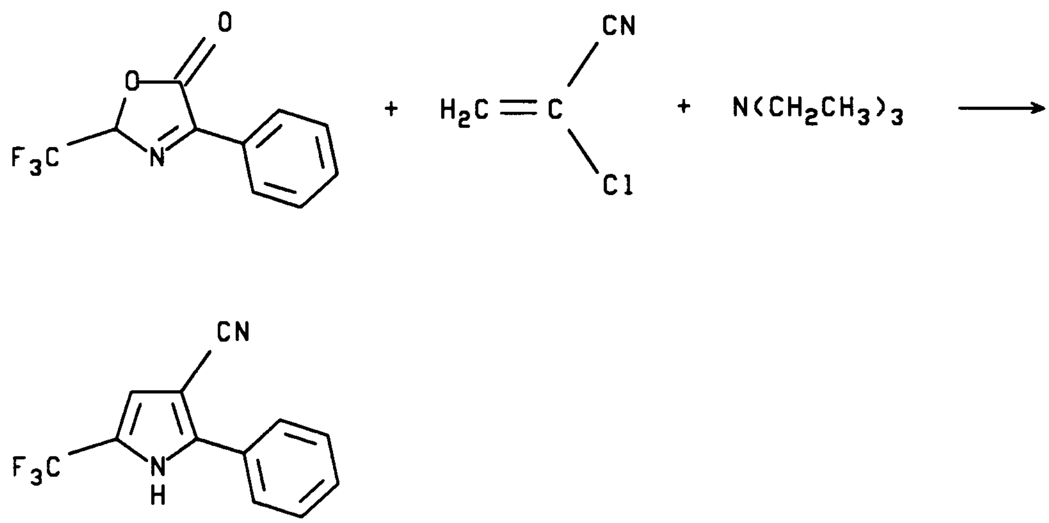

Triethylamine (4.5g, 0.044 mol) is added dropwise to a mixture of 4-phenyl-2-(trifluoromethyl)-5(2H)-oxazolone (10.0g, 0.043 mol), 2-chloroacrylonitrile (3.51g, 0.43 mol) and acetonitrile (50 mL). The temperature of the reaction mixture rises to 65 °C and carbon dioxide gas evolves during the addition. After the addition is complete the reaction mixture is heated at reflux temperature for one hour, cooled to room temperature and poured into water (150 mL). The solids are collected by filtration, air-dried and dried under vacuum at 60 °C to give the title compound as a white solid (10.05g, 97%). Identified by NMR spectral analyses.

The process described affords a nearly quantitative yield of the product pyrrole using stoichiometric amounts of reagents and short reaction time.

**EXAMPLE 2**

**Preparation of 2-Phenyl-5-(trifluoromethyl)pyrrole-3-carbonitrile without added base**

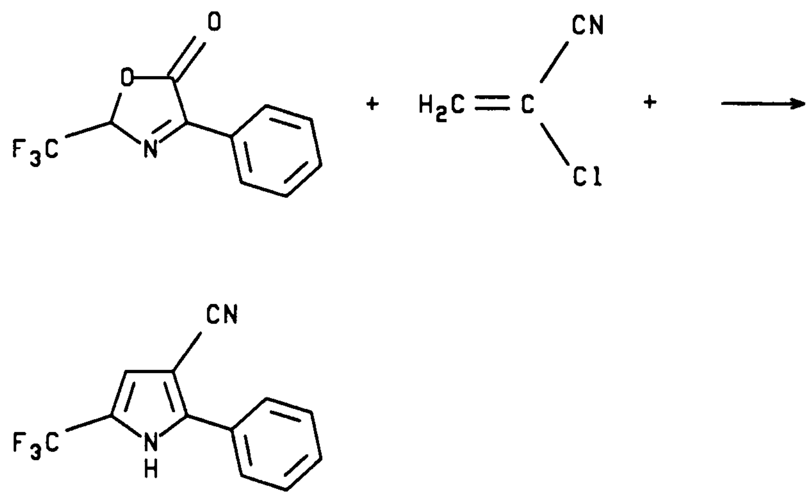

4-Phenyl-2-(trifluoromethyl)-5(2H)-oxazolone (13.5g, 0.059 mol), 2-chloroacrylonitrile (26 mL, 0.295 mol) and acetonitrile (250 mL) are heated at reflux temperature for 52 hours. The reaction mixture is then cooled to room temperature and concentrated in vacuo to give a solid. Recrystallization of the solid from toluene gives the title product as a yellow solid (10.1g, 73%). Identified by NMR spectral analyses.

The procedure without added base gives only a 73% yield of the title product despite the use of a large excess of 2-chloroacrylonitrile and a long reaction time.

**EXAMPLE 3**

**Preparation of Methyl 2-(p-chlorophenyl)-5-(trifluoromethyl)pyrrole-3-carboxylate**

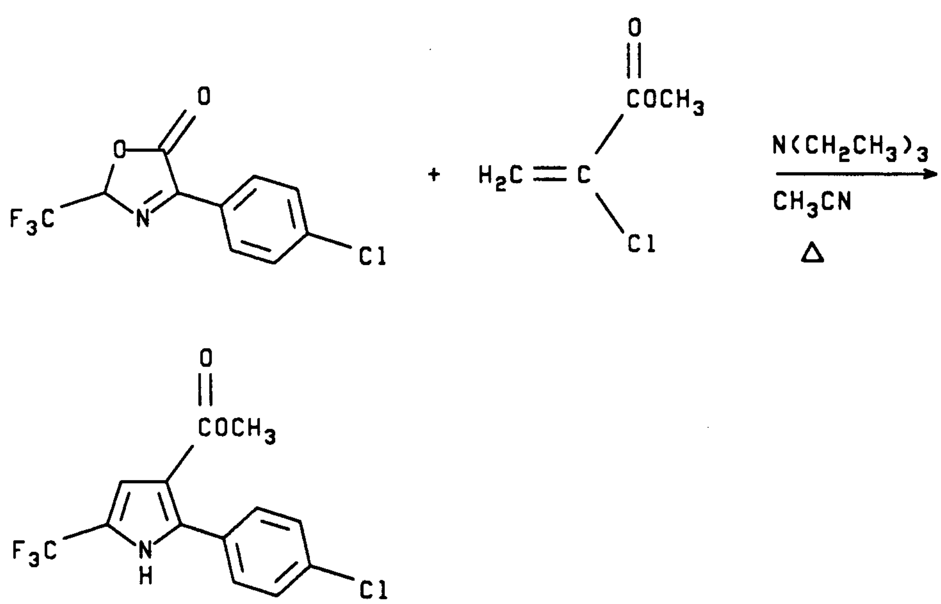


Triethylamine (5.58g, 0.055 mol) is added dropwise to a mixture of 4-(p-chlorophenyl)-2-(trifluoromethyl)-5(2H)-oxazolone (15.6g, 0.054 mol), methyl α-chloroacrylate (6.52.g, 0.054 mol) and acetonitrile (50 mL). The reaction mixture heats up to 55°C during the addition. After the addition is complete, the reaction mixture is heated at reflux temperature for one hour, cooled to room temperature and filtered. The filtrate is poured into an ether/water mixture and extracted with ether. The combined organic extracts are dried over anhydrous magnesium sulfate and concentrated in vacuo to give a black residue. Recrystallization of the residue from hexane gives the title compound as an orange solid (8.25g, 50.2%). Identified by NMR spectral analyses.

## EXAMPLE 4

### Preparation Using Non-Polar Solvent Instead of Polar Solvent

This example shows the necessity of using a polar solvent. Surprisingly, the base-catalyzed reaction when run in the presence of a non-polar solvent such as toluene does not result in the desired pyrrole.

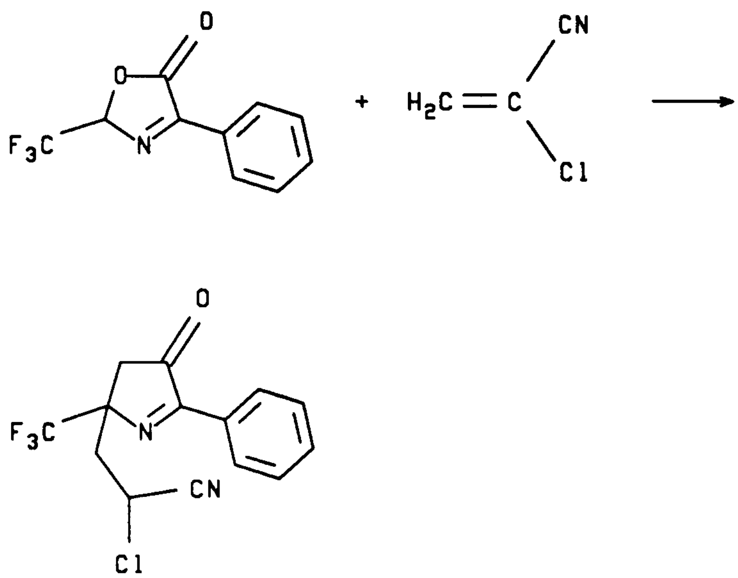

2-chloroacrylonitrile (3.85g, 0.044 mol) is added to a 0°C mixture of 4-phenyl-2-(trifluoromethyl)-5(2H)-oxazolone (10.0g, 0.044 mol) and toluene (50 mL). Triethylamine (4.49g, 0.044 mol) is added dropwise to the reaction mixture and the temperature rises to 25°C. When the addition is complete, water is added to the reaction mixture and the layers are separated. The organic layer is dried over anhydrous magnesium sulfate and concentrated in vacuo to give alpha-chloro-5-oxo-4-phenyl-2-(trifluoromethyl)-3-oxazoline-2-propionitrile as an amber liquid (8.37g, 60%). Identified by NMR spectral analyses.

## EXAMPLE 5

This example demonstrates that with the addition of a base and a polar solvent such as acetonitrile to the product formed in Example 4 the desired pyrrole is formed.

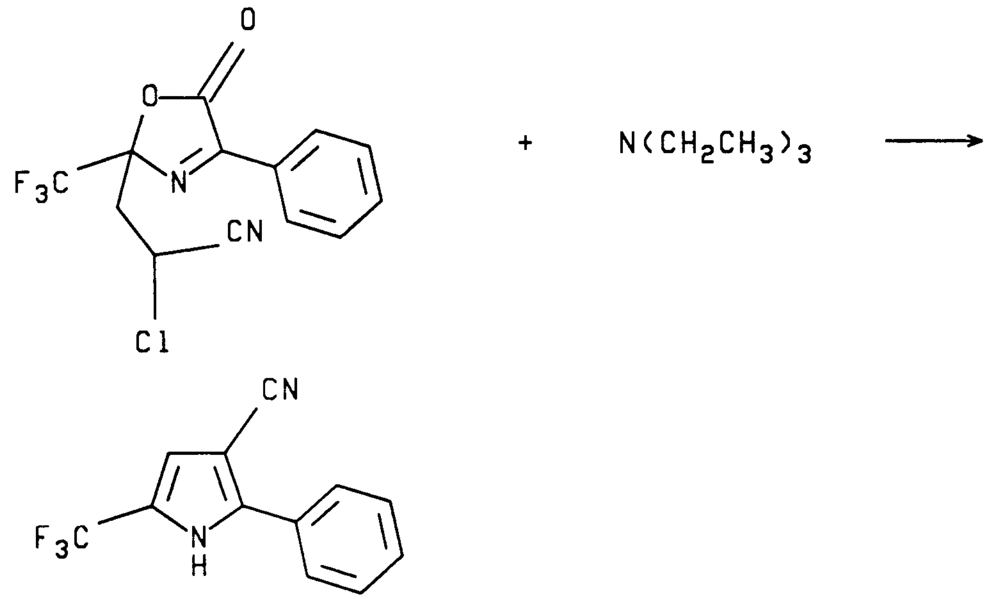

Triethylamine (0.077g, 0.76 mmol) is added dropwise to a refluxing mixture of alpha-chloro-5-oxo-4-phenyl-2-(trifluoromethyl)-3-oxazoline-2-propionitrile (0.24g, 0.76 mmol) and acetonitrile (25 mL). The reaction mixture is refluxed for 2 hours, cooled to room temperature and poured into water. The solids are collected by filtration and dried to give the title compound as a white solid (0.16g, 88%). Identified by NMR spectral analyses.

## EXAMPLE 6

### Solvent and base effects upon 2-aryl-5-(trifluoromethyl)pyrrole formation

The effects of solvent and base changes on the synthesis of 2-phenyl-5-(trifluoromethyl)pyrrole-3-carbonitrile from 4-phenyl-2-(trifluoromethyl)-5(2H)-oxazolone and 2-chloroacrylonitrile are shown in Table I. The percentages shown represent area percent determined by HPLC analysis of the reaction mixture after refluxing for 1/2 hour.

**TABLE I**

**Solvent and Base Effects**

| Solvent | Base | % Yield Arylpyrrole |
|---|---|---|
| Toluene | Triethylamine | 11 |
| Toluene | Pyridine | 8 |
| Tetrahydrofuran | Triethylamine | 39 |
| Ethanol | Triethylamine | 80 |
| Acetonitrile | Triethylamine | 96 |
| Acetonitrile | - | 4* |
| Dimethylformamide | Pyridine | 100 |
| Dimethylformamide | Sodium carbonate | 74 |
| Dimethylformamide | Triethylamine | 100 |

* 1 hour refluxing

## EXAMPLE 7

### Base effects in 2-aryl-5-(trifluoromethyl)pyrrole formation

Base effects on the synthesis of 2-aryl-5-(trifluoromethyl)pyrroles are shown in Table II. Acetonitrile is the solvent in all reactions.

TABLE II

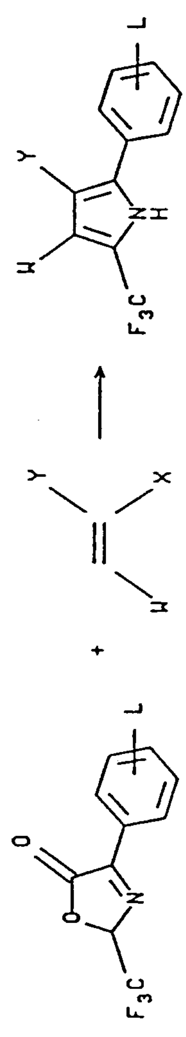

| W | L | Y | X | Equivalents of triethylamine | Time | % Yield |
|---|---|---|---|---|---|---|
| H | 4-Cl | CN | Cl | 0 | 78[a] | 79 |
| H | 4-Cl | CN | Cl | 1 | 1/2[a] | 96 |
| $CF_3$ | H | $CO_2C_2H_5$ | Br | 0 | 24[a] | 2 |
| $CF_3$ | H | $CO_2C_2H_5$ | Br | 1 | 2[b] | 20 |
| H | 4-Cl | $CO_2CH_3$ | Cl | 0 | 4 1/2[a] | 27 |
| H | 4-Cl | $CO_2CH_3$ | Cl | 1 | 1[a] | 50 |

a Hours reaction mixture is refluxed.
b Hours reaction mixture is stirred at room temperature.

**EXAMPLE 8**

**Solvent effects in 2-aryl-5-(trifluoromethyl)pyrrole formation**

Solvent effects in the formation of 2-(p-chlorophenyl-5-(trifluoromethyl)pyrrole-3-carbonitrile from 4-(p-chlorophenyl)-2-(trifluoromethyl)-5(2H)-oxazolone and 2-chloroacrylonitrile are shown in Table III.

TABLE III

| Solvent effects in 2-aryl-5-(trifluoromethyl)pyrrole formation | | | |
|---|---|---|---|
| Solvent | Equivalents of 2-Chloroacrylonitrile | Time[a] | % Yield |
| Nitromethane | 10 | 21 | 14 |
| Toluene | 10 | 17 | 17 |
| Trifluoroacetic Anhydride | 10 | 17 | 17 |
| Acetonitrile | 10 | 17 | 46 |

[a] Hours reaction mixture is refluxed

The results of these experiments show that even with polar solvents and long times, yields are generally poor in the absence of added base.

**EXAMPLE 9**

**Preparation of 4,4,4-Trifluorocrotononitrile (E)- and(Z)-**

Trifluoroacetaldehyde (47.4g, 0.48 mol), generated by addition of 1-ethoxy-2,2,2-trifluoroethanol (77.6g, 0.48 mol) to polyphosphoric acid (300 mL) heated to 150-180 °C, is swept with nitrogen over 2 hours into a slurry of (triphenylphosphoranylidene) acetonitrile (97.3g, 0.32 mol) in ether (400 mL). The reaction mixture is stirred overnight under nitrogen. After filtering off the solid triphenylphosphine oxide, the ether is distilled off to obtain an orange solution. Bulb to bulb distillation of the solution affords 2 fractions of the title compound as a clear colorless oil (14.3g, 37%). Fraction 1 (bp 20-40 °C , 14mm) contains a 6:1 mixture of (E):(Z). Fraction 2 (bp 40-50 °C, 14mm) contains a 2:1 mixture of (Z):(E). The fractions are identified by NMR analyses.

**EXAMPLE 10**

**Preparation of 2,3-Dibromo-4,4,4-Trifluorobutyronitrile**

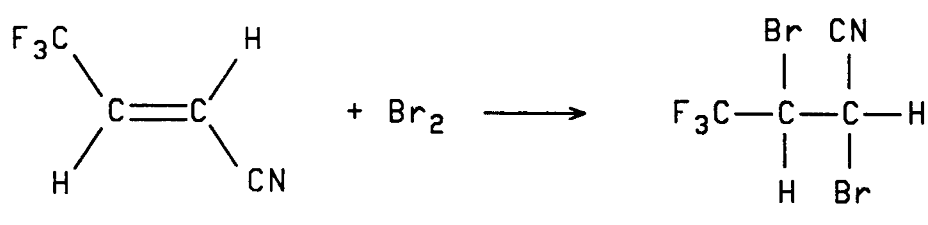

(E) and (Z)

Bromine (4.57g, 0.029 mol) is added over a 5 minute period to a solution of 4,4,4-trifluorocrotononitrile (3.46g, 0.029 mol) and carbon tetrachloride (70 mL). The resulting dark red solution is refluxed for 5 hours, then water (70 mL) is added to terminate the reaction. The organic layer is separated, washed sequentially with water, 5% sodium thiosulfate solution and water, dried over anhydrous magnesium sulfate, filtered and concentrated in vacuo to yield the title product as a yellow oil (6.1g, 76%), identified by NMR spectral analyses.

Following the procedure of example 10, but substituting the appropriately substituted 1,1,1-trifluoro-2-propene for 4,4,4-trifluorocrotononitrile yields the following compounds.

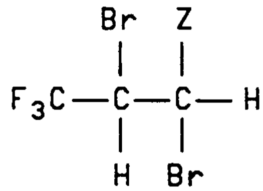

| Z | State |
| --- | --- |
| 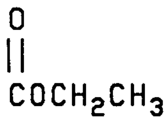 | colorless oil |
| 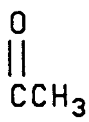 | yellow oil |
| 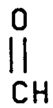 | yellow oil |

**EXAMPLE 11**

**Preparation of 2-Bromo-4,4,4- Trifluorocrotononitrile (E)- and (Z)-**

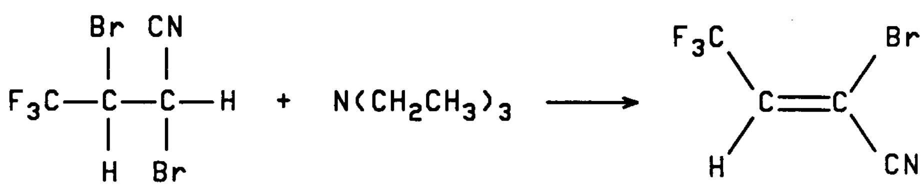

(E) and (Z)

Triethylamine (3.7g, 0.037 mol) is added dropwise to a solution of 2,3-dibromo-4,4,4-trifluorobutyronitrile (5.32g, 0.023 mol) and tetrahydrofuran (100 mL). A voluminous white precipitate forms during the addition. Stirring is continued for 1 hour, then the solid is filtered off and washed with tetrahydrofuran. The tetrahydrofuran is distilled off to give a dark brown oil. Bulb to bulb distillation (15 mm, 24 °C) of the oil yields the title product as a clear colorless liquid (3.1g, 82%). NMR spectral analyses identifies a 6:1 mixture of E:Z .

Following the procedure described in example 11, but using the appropriately substituted 1,2-dibromo-3,3,3-trifluoropropane for 2,3-dibromo-4,4,4-trifluorobutyronitrile yields the compounds shown below.

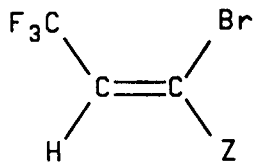

(E) and (Z)

| Z | State |
| --- | --- |
| 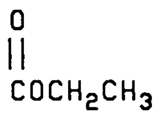 | clear yellow oil |
| 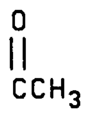 | red oil |

## EXAMPLE 12

### Preparation of 2-(p-chlorophenyl)-4,5-bis(trifluoromethyl)pyrrole-3-carbonitrile

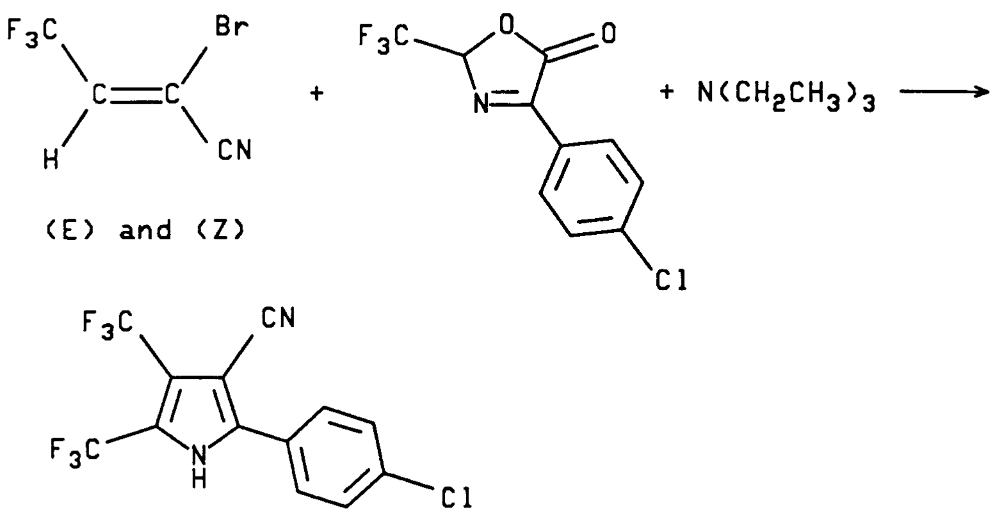


4-(p-Chlorophenyl)-2-(trifluoromethyl)-2-oxazolin-5-one (2.0g, 7.59 mmol) and 2-bromo-4,4,4-trifluorocrotononitrile (0.81g, 4.05 mmol) are dissolved in acetonitrile (10 mL). To the resulting yellow solution triethylamine (0.45g, 4.46 mmol) is added dropwise while the reaction flask is cooled with a water bath. After stirring at 25 °C overnight, the reaction mixture is poured into water. The aqueous layer is extracted with ethyl acetate and the combined organic extracts are washed sequentially with water, 5% sodium thiosulfate solution and brine, dried over anhydrous magnesium sulfate and concentrated in vacuo to give a yellow solid. The solid is chromatographed using silica gel and eluted with hexane/ethyl acetate 3:1 to yield the title compound as yellow crystals (1.26g, mp 208 °C).

Following the procedure of example 12, but substituting 3-bromo-5,5,5-trifluoro-3-penten-2-one for 2-bromo-4,4,4-trifluorocrotononitrile yields 2-(p-chlorophenyl)-4,5-bis(trifluoromethyl)pyrrol-3-yl methylketone

as yellow crystals.

## EXAMPLE 13

### Preparation of Ethyl 2-(p-chlorophenyl)- 4,5-bis (trifluoromethyl)pyrrole-3-carboxylate

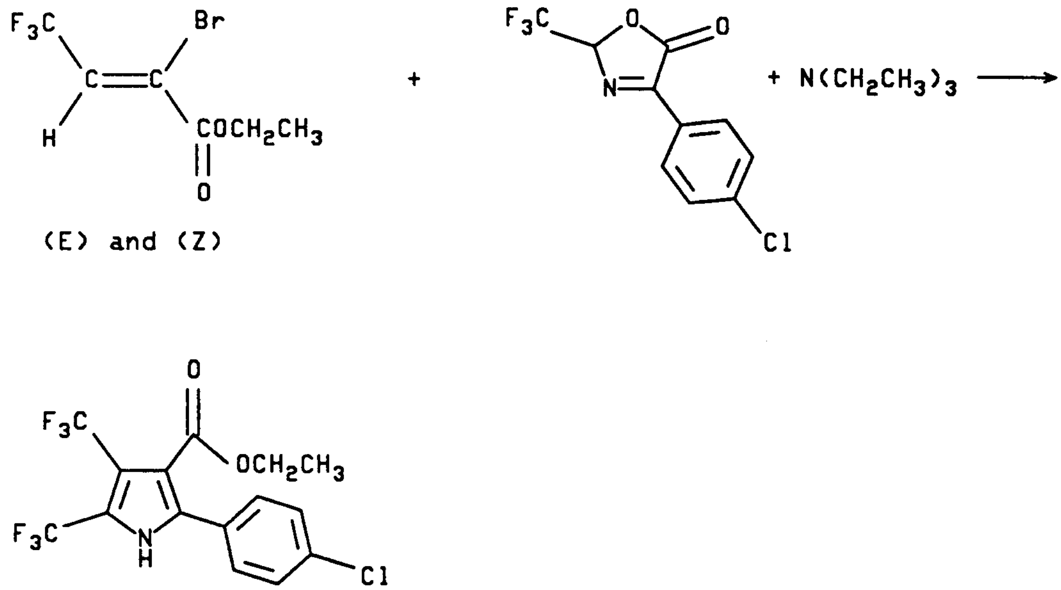

(E) and (Z)

Triethylamine (1.1g, 0.011 mol) is added dropwise to a solution of 4-(p-chlorophenyl)-2-(trifluoromethyl)-2-oxazolin-5-one (2.64g, 0.01 mol) and acetonitrile (25 mL). After stirring for 10 minutes at room temperature, a solution of ethyl 2-bromo-4,4,4-trifluorocrotonate (2.47g, 0.01 mol) and acetonitrile (1 mL) is added dropwise to the reaction mixture. The reaction mixture is stirred at room temperature for 2 hours then poured into water and extracted with ethyl acetate. The combined organic extracts are washed sequentially with water and brine, dried over anhydrous magnesium sulfate and concentrated in vacuo to give an orange oil which solidifies upon standing. Trituration with hexanes gives the title product as a yellow solid (2.36g, 61.3%, mp 138°-140°c).

**EXAMPLE 14**

**Preparation of 2-(3,4-dichlorophenyl)-4,5-bis(trifluoromethyl)pyrrole-3-carbonitrile**

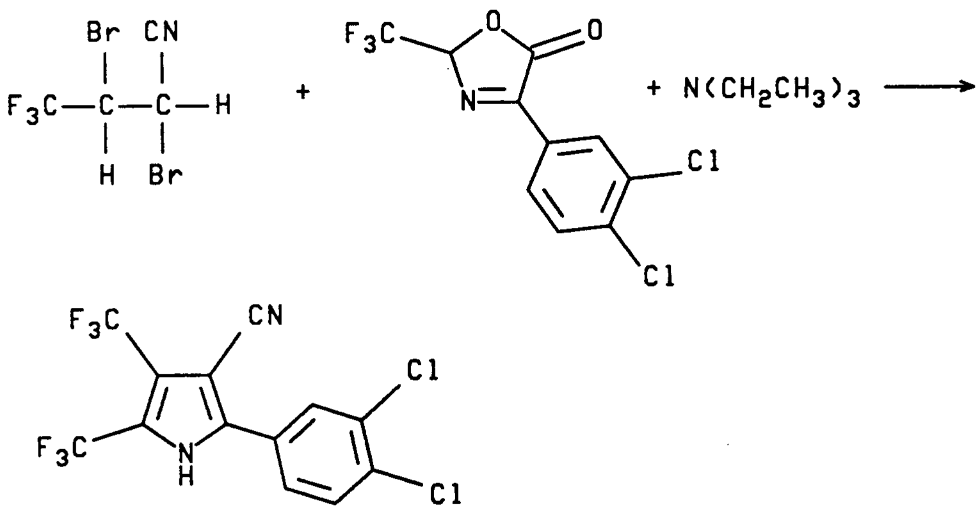

Triethylamine (0.59g, 5.83 mmol) is added dropwise to a solution of 2,3-dibromo-4,4,4-trifluorobutyronitrile (1.63g, 10.2 mmol) and tetrahydrofuran (20 mL). Stirring is continued for 15 minutes then a solution of 4-(3,4-dichlorophenyl)-2-(trifluoromethyl)-2- oxazlin-5-one (2.6g, 8.72 mmol), triethylamine (0.88g, 8.7 mmol) and acetonitrile (5 ml) is added dropwise to the reaction mixture. The reaction mixture is stirred overnight at room temperature, poured into water and extracted with ethyl acetate. The combined organic extracts are washed sequentially with water and brine, dried over anhydrous magnesium sulfate and concentrated in vacuo to give a brown oil. Chromatography of the oil using silica gel and hexanes/ethyl acetate 3:1 yields the title product as a pale yellow solid (1.49g, 67%, mp 205-208 °C).

**Claims**

**1.** A process for the preparation of a first compound having the structural formula: wherein

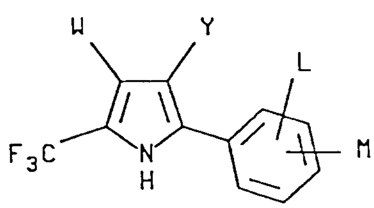

(I)

| | |
|---|---|
| W is | $C_1$-$C_4$ alkyl, $CF_3$ or H; |
| Y is | CN, $NO_2$ or $CO_2R$; |
| R is | $C_1$-$C_4$ alkyl; |
| L is | H, F, Cl or Br; |
| M is | H, F, Cl, Br, I, $CF_3$, $NO_2$ or $OR_1$; and |
| $R_1$ is | $C_1$-$C_3$ alkyl or $C_2F_4H$ which comprises reacting a second compound having the structure |

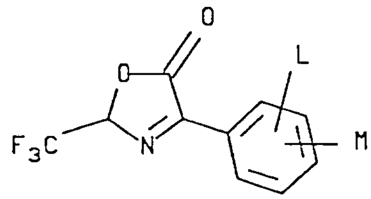

(II)

wherein L and M are as described above with at least 1 molar equivalent of a third compound having the structure

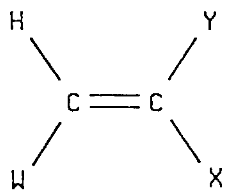

(III)

wherein W and Y are as described above, X is Cl, Br, I or

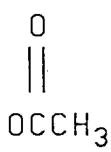

and the cis and trans isomers thereof in the presence of at least 1 molar equivalent of a base selected from an alkali metal carbonate, $C_1$-$C_4$ trialkylamine or pyridine and a polar solvent selected from acetonitrile, dimethylformamide, dimethylsulfoxide, ethanol, methanol or isopropanol, to form said first compound.

2. The process according to Claim 1 wherein the base is triethylamine.

3. The process according to Claim 1 wherein the polar solvent is acetonitrile.

4. The process according to Claim 1 wherein the temperature of the reaction mixture is 20° to 180°C.

5. A compound having the structure:

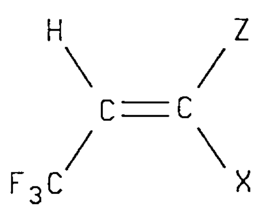

wherein
X is Cl, Br or I;
Z is CN or $CO_2R$;
R is $C_1$-$C_4$ alkyl; and the cis and transisomers thereof.

**6.** The compound according to claim 5, wherein
X is Cl or Br; and Z is CN.

**7.** The compound according to claim 5, 2-bromo-4,4,4-trifluorocrotononitrile.

**8.** A method for the preparation of a first compound having the structure:

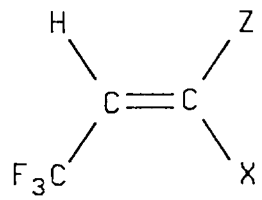

wherein X and Z are as defined in Claim 5;
and the cis and trans isomers thereof which comprises reacting a second compound having the structure

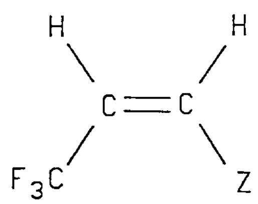

wherein Z is as described above; and the cis and trans isomers thereof with at least 2 molar equivalents of a halogenating agent in the presence of a solvent to yield an intermediate compound having the structure

$F_3CCHXCHXZ$

wherein X and Z are as described above and reacting said intermediate with at least one molar equivalent of a base in the presence of solvent to form said first compound.

**9.** The method according to Claim 8, wherein the base is triethylamine, pyridine or sodium carbonate; the halogenating agent is bromine or chlorine; and the reaction solvent is tetrahydrofuran, carbon tetrachloride or ether.

**Patentansprüche**

**1.** Ein Verfahren zur Herstellung einer ersten Verbindung mit der Strukturformel:

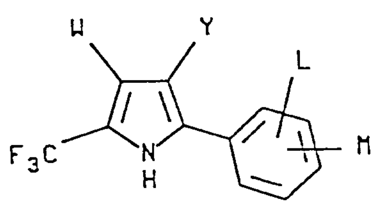

(I)

worin
W $C_1$-$C_4$-Alkyl, $CF_3$ oder H;
Y CN, $NO_2$ oder $CO_2R$;

R $C_1$-$C_4$-Alkyl;
L H, F, Cl oder Br;
M H, F, Cl, Br, J, $CF_3$, $NO_2$ oder $OR_1$ und
$R_1$ $C_1$-$C_3$-Alkyl oder $C_2F_4H$ ist, welches die Umsetzung einer zweiten Verbindung der Struktur

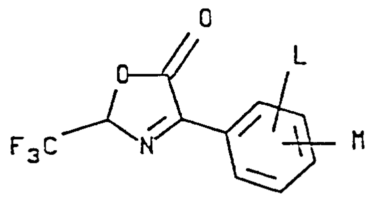

(II)

worin L und M wie oben beschrieben sind, mit mindestens einem Moläquivalent einer dritten Verbindung der Struktur

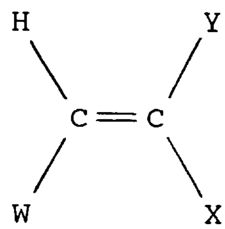

(III)

worin W und Y wie oben beschrieben sind, X Cl, Br, J oder

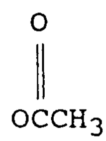

ist sowie deren cis- und trans-Isomeren in Gegenwart von mindestens einem Moläquivalent einer Base, ausgewählt aus einem Alkalimetallcarbonat, $C_1$-$C_4$-Trialkylamin oder Pyridin und einem polaren Lösungsmittel, ausgewählt aus Acetonitril, Dimethylformamid, Dimethylsulfoxid, Ethanol, Methanol oder Isopropanol, um diese erste Verbindung zu bilden, umfaßt.

**2.** Das Verfahren gemäß Anspruch 1, worin die Base Triethylamin ist.

**3.** Das Verfahren gemäß Anspruch 1, worin das polare Lösungsmittel Acetonitril ist.

**4.** Das Verfahren gemäß Anspruch 1, worin die Temperatur des Reaktionsgemischs 20° bis 180°C beträgt.

**5.** Eine Verbindung der Struktur:

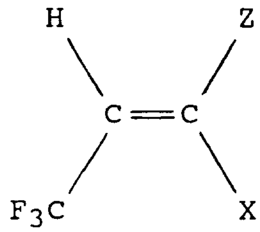

worin
X Cl, Br oder J;
Z CN oder $CO_2R$;
R $C_1$-$C_4$-Alkyl ist und deren cis- und trans-Isomere.

**6.** Die Verbindung gemäß Anspruch 5, worin X Cl oder Br und Z CN ist.

**7.** Die Verbindung gemäß Anspruch 5, 2-Brom-4,4,4-trifluorcrotonitril.

**8.** Ein Verfahren zur Herstellung einer ersten Verbindung mit der Struktur:

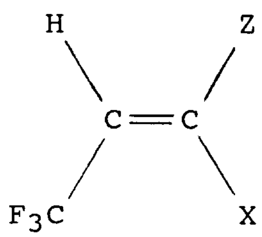

worin X und Z wie in Anspruch 5 definiert sind sowie deren cis- und trans-Isomeren, welches die Umsetzung einer zweiten Verbindung der Struktur

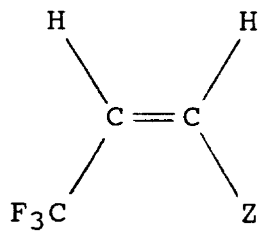

worin Z wie oben beschrieben ist, sowie deren cis- und trans-Isomeren mit mindestens 2 Moläquivalenten eines Halogenierungsmittels in Gegenwart eines Lösungsmittels unter Erhalt einer Zwischenverbindung der Struktur

$F_3CCHXCHXZ$

worin X und Z wie oben beschrieben sind und die Umsetzung dieser Zwischenverbindung mit mindestens einem Moläquivalent einer Base in Gegenwart eines Lösungsmittels zur Bildung der ersten Verbindung umfaßt.

**9.** Das Verfahren gemäß Anspruch 8, worin die Base Triethylamin, Pyridin oder Natriumcarbonat; das Halogenierungsmittel Brom oder Chlor und das Reaktionslösungsmittel Tetrahydrofuran, Tetrachlorkohlenstoff oder Ether ist.

## Revendications

**1.** Procédé de préparation d'un premier composé ayant la formule structurelle :

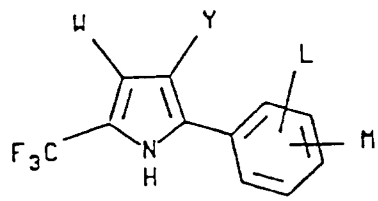

(I)

dans laquelle
W est un groupe alkyle en $C_1$-$C_4$, $CF_3$ ou un atome d'hydrogène;
Y est un groupe CN, $NO_2$ ou $CO_2R$;
R est un groupe alkyle en $C_1$-$C_4$;
L est un atome d'hydrogène, de fluor, de chlore ou de brome;
M est un atome d'hydrogène, de fluor, de chlore, de brome, d'iode, un groupe $CF_3$, $NO_2$ ou $OR_1$; et
$R_1$ est un groupe alkyle en $C_1$-$C_3$ ou un groupe $C_2F_4H$ qui comprend
la réaction d'un deuxième composé ayant la structure

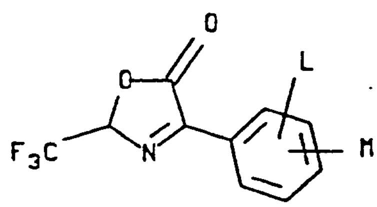

(II)

dans laquelle L et M sont tels que décrits ci-dessus avec au moins un équivalent molaire d'un troisième composé ayant la structure

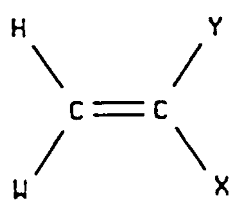

(III)

dans laquelle W et Y sont tels que décrits ci-dessus, X est un atome de chlore, de brome, d'iode ou le groupe

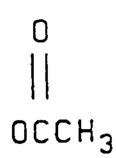

et des isomères cis et trans de ceux-ci en présence d'au moins un équivalent molaire d'une base choisie parmi un carbonate de métal alcalin, une trialkylamine en $C_1$-$C_4$ ou la pyridine, et un solvant polaire choisi parmi l'acétonitrile, le diméthylformamide, le diméthylsulfoxyde, l'éthanol, le méthanol ou

l'isopropanol pour former ledit premier composé.

2. Procédé selon la revendication 1 dans lequel la base est la triéthylamine.

3. Procédé selon la revendication 1 dans lequel le solvant polaire est l'acétonitrile.

4. Procédé selon la revendication 1, dans lequel la température du mélange réactionnel est comprise entre 20 et 180 °C.

5. Composé ayant la structure :

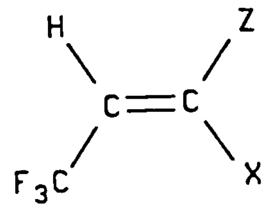

dans laquelle
X est un atome de chlore, de brome ou d'iode;
Z est un groupe CN ou $CO_2R$;
R est un groupe alkyle en $C_1$-$C_4$; et les isomères cis et trans de celui-ci.

6. Composé selon la revendication 5, dans lequel X est un atome de chlore ou de brome; et Z est un groupe CN.

7. Composé selon la revendication 5, qui est le 2-bromo-4,4,4-trifluorocrotononitrile.

8. Procédé de préparation d'un premier composé ayant la structure :

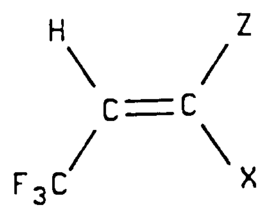

dans laquelle X et Z sont tels que définis dans la revendication 5;
et des isomères cis et trans de celui-ci, qui comprend la réaction d'un deuxième composé ayant la structure

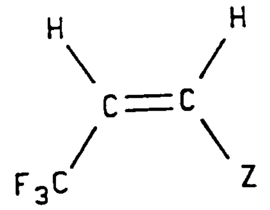

dans laquelle Z est tel que décrit ci-dessus; et les isomères cis et trans de celui-ci avec au moins deux équivalents molaires d'un agent d'halogénation en présence d'un solvant pour obtenir un composé intermédiaire ayant la structure

$F_3CCHXCHXZ$

dans laquelle X et Z sont tels que décrits ci-dessus et la réaction dudit intermédiaire avec au moins un équivalent molaire d'une base en présence de solvant pour former ledit premier composé.

**9.** Procédé selon la revendication 8, dans lequel la base est la triéthylamine, la pyridine ou le carbonate de sodium; l'agent d'halogénation est le brome ou le chlore; et le solvant de réaction est le tétrahydrofuranne, le tétrachlorure de carbone ou l'éther.